Europäisches Patentamt

European Patent Office

Office européen des brevets

(19)

(11) Publication number: 0 344 864
A2

## (12) EUROPEAN PATENT APPLICATION

(21) Application number: 89201381.4

(22) Date of filing: 31.05.89

(51) Int. Cl.⁴: C12N 15/00 , C12P 21/00 ,
C07K 15/00 , G01N 33/569 ,
A61K 39/29

(30) Priority: 03.06.88 US 202764

(43) Date of publication of application:
06.12.89 Bulletin 89/49

(84) Designated Contracting States:
AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: Kniskern, Peter J.
841 Patterson Drive
Lansdale, PA 19446(US)
Inventor: Hagopian, Arpi
771 Hartley Drive
Lansdale, PA 19446(US)
Inventor: Miller, William J.
232 Old Church Road
North Wales, PA 19454(US)
Inventor: Yamazaki, Shigeko
1279 Schwab Road
Hatfield, PA 19440(US)
Inventor: Ellis, Ronald W.
1407 Edgevale Road
Overbrook Hills, PA 19151(US)

(74) Representative: Hesketh, Alan, Dr. et al
European Patent Department Merck & Co.,
Inc. Terlings Park Eastwick Road
Harlow Essex, CM20 2QR(GB)

(54) Method for producing nonhyperglycosylated hepatitis B virus protein.

(57) The hepatitis B virus envelope proteins have been prepared in such a manner as to contain only polypeptide species in which the degree and nature of glycosylation is controlled and defined and in a such way that "hyperglycosylation" is eliminated. These proteins (and their component peptides) display the antigenic sites encoded by the S and preS domains of the HBV virion envelope open reading frame. Such proteins are useful for in vitro diagnostic systems and as vaccines for both the active and passive treatment or prevention of diseases and/or infections caused by the hepatitis B virus or other agents serologically related to the hepatitis B virus.

FIG. 1

# METHOD FOR PRODUCING NONHYPERGLYCOSYLATED HEPATITIS B VIRUS PROTEINS

## BACKGROUND OF THE INVENTION

Hepatitis B virus (HBV) is the infectious agent responsible for several varieties of human liver disease. Many individuals who are infected by HBV suffer through an acute phase of disease, which is followed by recovery. However, a percentage of infected individuals fail to clear their infection, thereby becoming chronic carriers of the infection. HBV infection is endemic in many parts of the world, with a high incidence of infection occurring perinatally from chronically infected mothers to their newborns who themselves often remain chronically infected. The number of chronic carriers worldwide has been estimated at over three hundred million. From this pool of carriers, hundreds of thousands die annually from the long-term consequences of chronic hepatitis B (cirrhosis and/or hepatocellular carcinoma).

The hepatitis B delta virus is an agent which, during coinfection with HBV, is responsible for an acute fulminating disease with a generally fatal resolution. The delta virus does not encode (from its own genetic material) proteins which serve as the virion envelope; rather, the virus encapsulates with the envelope proteins encoded by the coinfecting HBV, thereby sharing a close structural and immunologic relationship with the HBV proteins which are described below. It is unknown at this time whether other infectious agents share similar relationships with HBV. However it is clear that proteins with expanded breadth of serologic reactivity or enhanced immunogenic potency would be useful in systems for diagnosis or prevention (or treatment) of diseases (or infections) by a class of agents with even slight or partial antigenic cross-reactivity with HBV.

The HB virion is composed of two groups of structural proteins, the core proteins and the envelope or surface proteins. In addition to being the major surface proteins of the virion, i.e., Dane particle, the envelope proteins are the sole constituents of Australia antigen, or 22 nm particles. These envelope proteins are the translational products of a large viral open reading frame (ORF) encoding 389 amino acids (aa). This ORF is demarcated into three domains, each of which begins with an ATG codon that is capable of functioning as a translational initiation site in vivo. These domains are referred to as preS1 (108 aa), preS2 (55 aa), and S (226 aa) in their respective 5'-3' order in the gene. Thus, these domains define three polypeptides referred to as S or HBsAg (226 aa), preS2 + S (281 aa), and preS1 + preS2 + S (389 aa), also referred to as p24/gp27, p30/gp33/gp36 and p39/gp42 respectively.

The envelope proteins of HBV are glycoproteins with carbohydrate side chains (glycans) attached by N-glycosidic linkages to defined peptide recognition sites, [Heermann et al., J. Virol. 52, 396 (1984) and Stibbe et al., J. Virol. 46, 626 (1983)]. Thus the HBV polypeptides produced during natural infection comprise the species p24/gp27 (the S polypeptide and its glycosylated derivative), gp33/gp36 (the preS2 + S polypeptide glycosylated in the preS2 domain only and the same polypeptide glycosylated in the S as well as the preS2 domain), and p39/gp42 (the preS1 + preS2 + S peptide and its derivative glycosylated in the preS1 domain). Currently available plasma-derived vaccines are composed of proteins containing virtually only the S domain (comprising the p24 monomer and its glycosylated derivative gp27), while yeast-derived vaccines successfully developed to date are composed exclusively of the S polypeptide (comprising exclusively the nonglycosylated p24 species).

The 22 nm particles, or HB surface antigen (HBsAg) particles, have been purified from the plasma of chronic carriers. In terms of their plasma being particle-positive, these chronic carriers are referred to as HBs$^+$. If infected persons have mounted a sufficient immune response, they can clear the infection and become HBs$^-$. In terms of their formation of antibodies to HBs, these individuals are denoted anti-HBs$^+$. In this way, anti-HBs$^+$ is correlated with recovery from disease and with immunity to reinfection with HBV. Therefore, the stimulation or formation of anti-HBs by HB vaccines has been expected to confer protection against HBV infection.

This hypothesis has been testable experimentally. Outside of man, the chimpanzee is one of the few species which is fully susceptible to HBV infection, as reflected in quantifiable markers such as HBs$^+$ and elevated serum levels of liver enzymes. Chimpanzees have been vaccinated with three doses of purified HBsAg particles and then challenged intravenously with infectious HBV. While mock-vaccinated animals have shown signs of acute HBV infection, the HBsAg-vaccinated animals have been protected completely from any signs of infection. Therefore, in this experimental system, HBsAg particles, composed of gp27 and p24, have been sufficient to induce protective immunity. Spurred by these observations, several manufacturers have produced HB vaccines composed of HBsAg particles.

Recently, several independent lines of evidence have suggested that the preS sequences may be important in conferring immunity to HBV. The immune elimination of preS antigens during the course of viral infection appears prognostic for viral clearance and abrogation of infection [Budkowska et al., Ann. Inst. Past./Immun., 136D: 56-65, (1985)]. During acute hepatitis B infection, antibodies to the preS domains often arise earlier than antibodies to S [Petit et al., Mol. Immun., 23:511-523, (1986)]. In inbred mice, the immune responses to S and preS appear to be regulated independently, and the presence of the preS domain can influence the immune response to S [Milich et al., Proc. Nat. Acad. Sci. USA, 82:8168-8172, (1985), J. Immunol., 137:315-322 (1986); Neurath et al., J. Med. Virol., 17:119-125, (1985)]. Furthermore, antibodies to the preS domain neutralize viral infectivity in vitro [Neurath et al., Vaccine, 4:35-37,(1986)], and preS antigens protect immunized chimpanzees against HBV infection [Itoh et al., Proc. Nat. Acad. Sci. USA, 83:9174-9178, (1986)]. In light of these observations and because, as discussed below, of the utility of recombinant yeast in producing HB vaccines [Hilleman et al., Vaccine, 4:75-76, (1986)], we have formulated experimental preS-containing HB vaccines from recombinant Saccharomyces cerevisiae.

In order to expand the available supply of HB vaccines, manufacturers have turned to recombinant DNA technology to mediate the expression of viral envelope proteins. Among microbial systems, Escherichia coli and S. cerevisiae have been used most commonly for the expression of many recombinant-derived proteins. Numerous attempts to express immunologically active HBsAg particles in E. coli have been unsuccessful. However, S. cerevisiae has shown great versatility in its ability to express immunologically active HBsAg particles. These particles, when formulated into a vaccine, have proven capable of fully protecting chimpanzees against challenge with live HBV of diverse serotypes. Furthermore, yeast-derived S particles are also immunologically active and as effective in human clinical trials as plasma-derived HBsAg [Stevens et al., JAMA, 257: 2612-2616 (1987)]. Therefore, the utility of S. cerevisiae as a host species for directing the synthesis of recombinant HBsAg is established firmly. In addition, expression of human therapeutic agents and vaccines in yeast can be very useful for product development, since yeast is free of endotoxin, is nonpathogenic to man, can be fermented to industrial scale, and lacks many of the safety concerns which surround the use of continuous mammalian cell lines (many of which are virally transformed, may be tumorigenic in mice and all of which contain protooncogenes).

S. cerevisiae (bakers' yeast) is a eukaryote which is capable of synthesizing glycoproteins. Protein glycosylation in yeast has been the subject of numerous recent review articles [notably: Kukuruzinska et al., Ann. Rev. Biochem, (1987) 56, 915-44; Tannen et al., BBA, (1987) 906, 81-99]. This glycosylation or addition of glycans to appropriate receptor amino acids (aa) on the polypeptide occurs either at specific serine (Ser) or threonine (Thr) residues (O-linked) or at specified asparagine (Asn) residues (N-linked). The specificity for O-linked addition at Ser or Thr residues is not clearly understood and is determined empirically on a case-by-case basis.

The signal sequence for N-linked glycosylation is well defined as either of the aa sequences Asn-X-Thr or Asn-X-Ser (wherein X is any amino acid). In addition to synthesizing many autologous, native, glycosylated proteins (among them being those called mannoproteins), yeast also are capable of glycosylating heterologous or foreign proteins expressed by recombinant technology (if the heterologous protein contains the appropriate glycosylation signal sequence for either N-linked or O-linked glycosylation).

The preS2 + S polypeptides, which are produced during natural infection contain no more than two "core" [ca. 3 kilodaltons (KD) in size] N-linked glycans, one in the S region and a second on the Asn at aa residue 4 of the preS2 domain. The recognition site in the S domain is not glycosylated in either Recombivax HB® or in recombinant preS2 + S synthesized in yeast. However, the site at aa residue 4 of the preS2 domain is recognized and glycosylated by yeast.

The preS1 domain contains an N-linked glycosylation site at aa residue 4 of the preS1 region and a potential site at aa residue 26 for serotype adw. It is obvious to those skilled in the art that arguments set forth for preS2 glycosylation also will follow for diverse sequences in the preS2 region as well as for those in the preS1 and S domains.

Yeast synthesizing recombinant preS2 + S add a "core" glycan which is similar to that added to the native polypeptide during viral infection. However, if the yeast host cell is "wild-type" for glycosylation (i.e., containing the full complement of enzymes required for native glycosylation which is the case for virtually all commonly used strains), a significant number of these glycans are extended with a large number of additional mannose residues in a manner identical to that employed by yeast in making its own structural mannoproteins. This extended addition of the glycan, when it occurs on a foreign gene product such as the preS2 + S polypeptide, is referred to as hyperglycosylation. It is obvious to those skilled in the art that arguments set forth for yeast also will extend to other host cells (e.g., insect, fungi, etc.) which may be subject to divergent glycosylation patterns.

Hyperglycosylation may be eliminated or glycosylation limited in HBV preS and S polypeptides by any of the following approaches which illustrate the present invention without, however, limiting the same thereto.

Firstly, N-linked hyperglycosylation may be prevented or limited during growth of a recombinant host through the presence in the growth medium of an exogenous agent (e.g., tunicamycin). Secondly, carbohydrate may be reduced or removed from HBV polypeptides, from recombinant or natural sources, either chemically (e.g. anhydrous trifluoromethanesulfonic acid or anhydrous hydrogen fluoride) or enzymatically (e.g., with N-glycanase, Endo-F or Endo-H) or physically (e.g. sonication). Thirdly, the recognition site for glycosylation may be changed or deleted by mutagenesis at the DNA level, such that core glycosylation and thereby hyperglycosylation as well is prevented. Such modified preS2 + S ORFs in which the glycosylation recognition sequence has been altered (directed by suitable promoters active in yeast) have been transformed into yeast host cells. The resultant preS2 + S polypeptides lack glycosylation. Fourthly, host cells may be identified which lack critical enzymes required for glycosylation. One such yeast strain has been identified (mnn9 mutant) which lacks a critical enzyme in the glycosylation pathway necessary for the elongation (hyperglycosylation) of the N-linked glycans; chemical studies indicate that this mutant makes mannoproteins without outer-chain mannose residues and containing only the "core" carbohydrate. The ORF for the preS2 + S polypeptide (directed by suitable promoters active in yeast) has been used to transform such mnn9 mutant yeast. The resulting preS2 + S polypeptide contains only "core" glycosylation and lacks hyperglycosylation.

These and other objects of the present invention will be apparent from the following description.

## OBJECTS OF THE INVENTION

Thus, it is an object of the present invention to provide expression vectors, host cells and processes for the production of HBV envelope proteins and/or their component polypeptides in an antigenic form with controlled and defined glycosylation patterns. A second object of this invention is to maintain the antigenic structure of the viral (native) peptide domains and to eliminate the unwanted reactivities directed to host antigens conferred by the presence of host-specific glycosylation patterns. Another object of this invention is to specify conditions for the scale-up of the growth of recombinant host cells, such that maximal yields of the envelope proteins may be attained in much larger volumes and in higher concentrations for the purification of such polypeptides. A further object of this invention is to define those controlled conditions in which the glycosylation patterns of the proteins can be altered post-synthesis. These and other objects of the present invention will be apparent from the following description.

## BRIEF DESCRIPTION OF THE DRAWINGS

Figure 1 shows diagramatically the preparation procedure of pYGpreS2S-1, the preS2 + S expression vector with a "native" N-linked glycosylation recognition site in the preS2 domain.

Figure 2 shows diagramatically a comparison of the preS2 + S ORF to those mutants in which the N-linked glycosylation recognition site is eliminated by either changing the native Asn at position 4 of the preS2 sequence domain to Gln ($Gln^4$ mutant) or by changing the native Thr at postion 6 of the preS2 domain to Ala ($Ala^6$ mutant).

## SUMMARY OF THE INVENTION

The HBV preS2 + S gene has been expressed at an optimized high yield in yeast. The protein, which is expressed and/or treated in a manner to control the extent of glycosylation and thereby diminish or eliminate hyperglycosylation, assembles into a form which displays the major antigenic sites encoded by the plasma-derived HBV preS2 + S protein, thereby highlighting the utility of yeast as a host for the expression of this gene product. This protein is useful for in vitro diagnostic systems and as a vaccine for the treatment and/or prevention of HBV-related infections.

## DETAILED DESCRIPTION OF THE INVENTION

The present invention is directed to a method for the preparation of HBV envelope proteins, which contain limited, controlled or defined glycosylation patterns and which are not hyperglycosylated.

Dane particles (serotype adw) were utilized as the source of HBV nucleic acid for the isolation of the

4

viral ORFs. It is obvious to those skilled in the art that this invention extends to the use of nucleic acid from HBV strains with other serologic reactivities which derive from viral genetic diversity. The endogenous polymerase reaction was employed in order to produce covalently-closed circular double-stranded DNA of the HBV genome from the nicked and gapped nucleic acid form that natively resides in the HB virion. The DNA was isolated, digested to completion with EcoRI, and cloned into the EcoRI site of pBR322, thus generating pHBV/ADW-1. The recombinant plasmids containing the HBV genome in a circularly permuted form at the EcoRI site of the PreS region were selected. The complete ORF encoding the 55 amino acids of the preS2 region and the 226 amino acids of the S region was constructed first by purifying the 0.8 kilobase pair (kbp) fragment obtained following digestion of pHBV/ADW-1 with EcoRI and AccI; this fragment encodes the preS2 + S polypeptide lacking only the initiation codon, the amino-terminal 3 aa, the carboxy-terminal 3 aa, and the translational terminator codon.

Oligonucleotides were synthesized and ligated to this fragment, converting it to a HindIII fragment containing a 10 bp yeast-derived non-translated 5' flanking sequence and the complete preS2 + S ORF. The sequence at the 3' flank of the preS2 + S ORF was chosen such that the termination codon directly abutted a natural HindIII site in the ADHI transcriptional terminator, thus creating a completely native yeast-derived junction without any additional intervening bases. It is obvious to those skilled in the art that for expression of preS2 + S, any suitable yeast-active transcriptional terminator may be substituted for ADHI.

The 5' flanking sequence for the construction (ACAAAACAAAA) was chosen to correspond to that for the non-translated leader (NTL) of the yeast gene GAP63 (GAP) [Holland, J. Biol. Chem., 225, 2596 (1980)] and is also a consensus for the GAP gene family. The construction was made in such a manner as to abut the NTL directly to the initiation codon of the preS2 + S ORF without the intervention of any additional bases. Therefore, it is obvious to those skilled in the art that, for expression of envelope polypeptides, the selection of NTL sequences extends to other sequences which result in suitable expression levels.

DNA sequence analysis revealed 2 base substitutions which resulted in aa differences from the preS2 + S sequence encoded by the DNA of pHBpreSGAP347/19T [Valenzuela et al., Biotechnology, 3(4), 317-320 (1985)]. In order to evaluate identical polypeptides for both constructions, these nucleotide substitutions, which were T instead of C at base 64 of the 846 bp ORF of HBV preS2 + S (encoding Phe rather than Leu) and C instead of A at base 352 (encoding His rather than Gln) were changed by site-directed mutagenesis [Zoller et al., Nucleic Acids Research 10:6487-6500 (1982)]. The encoded aa sequence for the optimized construction then was verified. It is obvious to those skilled in the art that this invention is not limited to this sequence and extends to any sequence wherein the DNA encodes a polypeptide with HBV antigenicity.

Following mutagenesis, the fragment described above was used to construct an expression cassette, as described previously [Kniskern et al., Gene, 46:135-141, (1986)], which was composed of: (a) ca. 1.1 kbp of the GAP491 promoter, (b) a 10 bp yeast-derived flanking sequence, (c) 846 base pairs of the HBV preS2 + S gene (serotype adw) lacking any viral flanking sequences, and (d) ca. 0.4 kbp of the yeast ADH1 terminator. This expression cassette was inserted into the yeast shuttle vector pC1/1 [Beggs, Nature, 275:104, (1978); Rosenberg et al., Nature, 312: 77, (1984)] to create plasmid pYGpreS2S-1 which was used to transform yeast strain CF42, generating a transformant hereafter called pF403. This transformant was established as a frozen stock for evaluation and subsequent experimentation. Parental strain CF42 was obtained as follows: a spontaneous ura3 mutation in yeast strain 2150-2-3 (L. Hartwell, U. of Washington) was selected [Boeke et al., Mol. Gen. Genet., 197:345-346, (1984)]. The resulting strain (MATa, ade1⁻, leu2-04⁻, ura3⁻, cir°) was diploidized by transforming with plasmid YCp50-HO [Jensen et al., P.N.A.S. USA, 80:3035-3039, (1983)]. The functional yeast gene HO allows cells to switch mating type. Thus, progeny from single cell transformants will be a mixture of both a and α mating types and will mate during colony growth. A diploid clonal isolate was cured of the plasmid and designated CF42 (MATa/α, ade1⁻, leu2-04⁻, ura3⁻). These transformants were established as frozen stocks for evaluation and subsequent experimentation.

Recombinant yeast from the pF403 frozen stocks was grown in YEHD medium [Carty et al., J. Industrial Micro., 2, 117-121, (1987)]. After growth to stationary phase, yeast cells were harvested. Lysates were prepared, resolved by sodium dodecyl-sulfate-polyacrylamide gel electrophoresis (SDS-PAGE), and immunoblotted with antibodies to HBsAg. Two major polypeptides were found with molecular weights of 30- and 34-kD in accord with the predicted molecular weight of the translational product of the preS2 + S ORF and its glycosylated derivative. An additional polydisperse (molecular weight ≥ 50-kD) glycopeptide band also was detected which corresponded to the population of hyperglycosylated species and which was immunoreactive with anti-yeast as well as anti-HBs sera. Furthermore, lysates of recombinant, but not parental, yeast were positive for preS2 + S by radioimmunoassay (RIA). Electron microscopic examination of partially purified yeast lysates showed high densities of typical 22 nm preS2 + S particles.

5

The yeast-derived promoter initiates transcription of the preS2 + S gene. Therefore, it is obvious to those skilled in the art that any yeast-active promoter sequence may be substituted for the GAP491 promoter. It is also obvious to those skilled in the art that a suitable assay system, e.g., immunoblot or RIA or enzyme-linked immunoassay (EIA), should be utilized in order to assay expression of preS2 + S polypeptides in this system, such that the time of harvesting of the culture for attaining a maximal yield can be optimized.

The GAP491 promoter has been useful for the expression in yeast of several foreign proteins, including HBsAg [Bitter et al., Gene, 32:263-274, (1984); Wampler et al., Proc. Nat. Acad. Sci. USA, 82:6830-6834, (1985)]. Based upon our previous results of expressing HBcAg to ca. 40% of soluble yeast protein (Kniskern et al., supra), we have used this promoter to drive the expression of preS2 + S antigen in suitable yeast host cells.

In order to control and define the glycosylation of recombinant yeast-expressed preS2 + S protein, the yeast expression plasmid (pYGpreS2S-1) containing the expression cassette described above also was used to transform S. cerevisiae strain KHY-107 (cir$^+$, ade1$^+$, leu2$^-$, mnn9$^-$) which was constructed as follows:

An $\alpha$ mating type strain CZ5/LB347-1C (mnn 9$^-$, SUCZ$^-$) (C. Ballou, U. of Calif.) was mated with the a type strain 2150-2-3 (leu2$^-$, adel$^-$) (L. Hartwell, U. of Washington) by mixing the strains on a YEHD agar plate (Carty et al., supra). To select for diploids, the mated strains were replica-plated onto minimal media without leucine (leu$^-$) and containing 2% sucrose as the sole carbon source. After isolating single colonies, the diploids were sporulated, and asci were dissected by standard techniques. The KHY-107 strain was isolated as a single spore and characterized as cir$^+$, adel$^+$, leu2$^-$, and mnn9$^-$ (by Schiff stain technique).

Transformed clones were selected on minimal medium (leu$^-$) containing 1M sorbitol. These cloned transformants were established as frozen stocks in 17% glycerol for subsequent evaluation and further experimentation.

The expression plasmid pYGpreS2S-1 also was used to transform KHY-107 (cir$^\circ$, ade1$^+$, leu2$^-$, mnn9$^-$), which was derived from strain KHY-107 (cir$^+$, ade1$^+$, leu2$^-$, mnn9$^-$) as described (Broach, G.R. "Methods in Enzymology", Vol. 101, part C, pg 307-325, 1987, Academic Press, N.Y.). Transformed clonal isolates were established as frozen stocks in 17% glycerol for subsequent evaluation and further experimentation.

Clones of transformed yeast [KHY-107(cir$^+$, ade1$^+$,leu2$^-$, mnn9$^-$)] containing the expression plasmid pYGpreS2S-1 were plated onto leu$^-$ selective agar plates containing 1M sorbitol, and incubated at 30$^\circ$ C for 2-3 days. These yeast were inoculated into 5-7 mL cultures of complex YEHD (Carty et al., supra) medium containing 1M sorbitol, and the cultures were incubated at 30$^\circ$ C with aeration for 12-18 hrs. Flasks containing 50 mL complex YEHD media with 1M sorbitol (hereafter called YEHDS) were inoculated from the above cultures (to an initial A$^{600}$ = 0.1) and were incubated at 30$^\circ$ C with shaking (350 rpm) for 48-72 hrs to a final A$^{600}$ of 10-16. Samples of 10 A$^{600}$ units were aliquoted into tubes, and the yeast cells were pelleted at 2000xg for 10 minutes. Samples either were assayed directly or stored frozen at -70$^\circ$ C. At the time of assay, the pellets were resuspended in 0.4 mL of phosphate-buffered saline (PBS) containing 2mM phenylmethyl sulfonyl fluoride (PMSF) and transferred to 1.5 mL Eppendorf tubes. Yeast cells were broken by: 1) the addition of 200-300 mg of washed glass beads (0.45 mm) and agitation on a vortex mixer for 15 min, 2) addition of TX-100 to 0.5%, 3) agitation on the vortex mixer for 2 minutes, and 4) incubation at 4$^\circ$ C for 10 minutes. Cellular debris and glass beads were removed by centrifugation at 2000xg for 10 minutes. The clarified supernatant fluid was removed and assayed for protein [by the method of Lowry et al., J. Biol. Chem., 193, 265, (1951)] and RIA specific for preS2 + S [Hansson et al., Infect. Immunol. 26: 125-130, (1979), Machida et al., Gastroenterology 86: 910-918, (1984)].

Five clones were evaluated in parallel and compared to an equivalent cell pellet from clone pF403 which was normalized to a value of 1.0 for reference. Typical relative values of antigen productivity for the five clones were obtained as listed in Example VII.

Clones of transformed yeast [KHY-107 (cir$^\circ$, ade1$^+$, leu2$^-$, mnn9$^-$)] containing the expression plasmid were plated onto leu$^-$ selective agar plates containing 1M sorbitol and incubate at 30$^\circ$ C for 2-3 days. These yeast were inoculated into 5-7 mL cultures of complex YEHDS media, and the cultures were incubated at 30$^\circ$ C with aeration for 12-18 hrs. Flasks containing 50 mL complex YEHDS media were inoculated from the above cultures (to an initial A$^{600}$ = 0.1) and were incubated at 30$^\circ$ C with shaking (350 rpm) for 48-72 hrs to a final A$^{600}$ of 10-16. Triplicate samples of 10 A$^{600}$ units were aliquoted into tubes, and the yeast cells were pelleted at 2000 xg for 10 min.

Samples either were assayed directly as described above or stored frozen at -70$^\circ$ C.

Five clones were evaluated in parallel and compared to clone pF403 which was normalized to a value of 1.0 for reference. Typical relative values of antigen productivity for the five clones were obtained as listed in Example VIII.

Immunoblot analysis of the preS2 + S polypeptide derived from all recombinant clones described above, in host cells with the mnn9 phenotype, showed two bands with apparent molecular sizes of 30- and 34-kD. The polydisperse (molecular weight > 50-kD) hyperglycosylated species were not detected with either anti-yeast or anti-HBs sera.

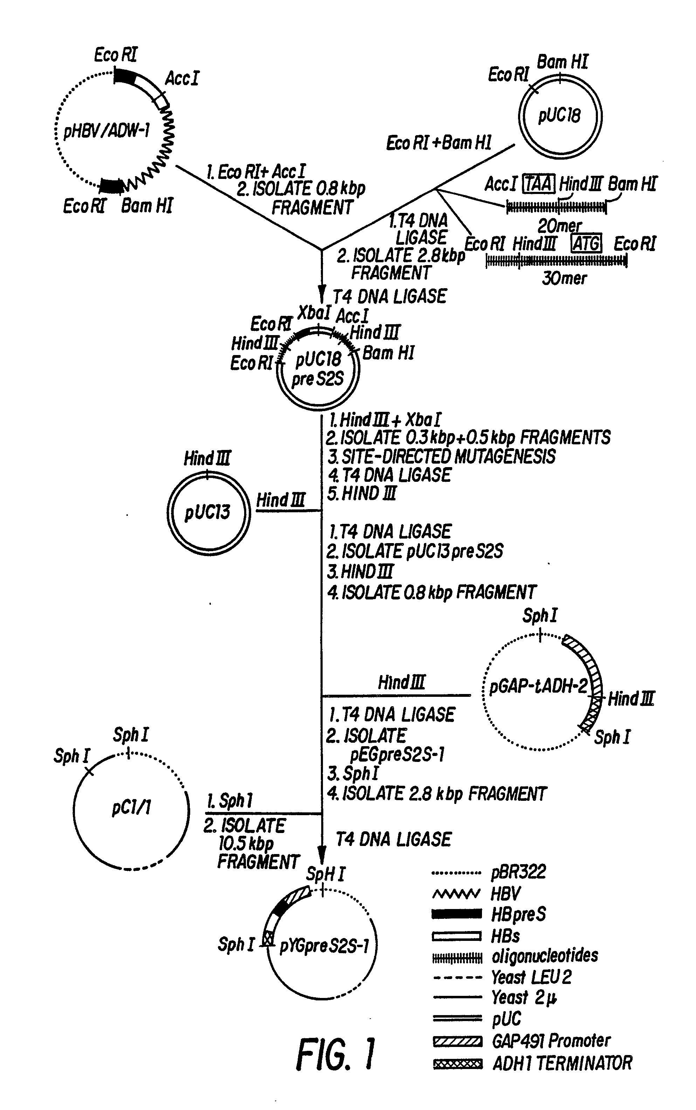

In order to provide for an expression vector in which the intrinsic nature of the expressed protein defines the control of glycosylation of the HBV preS2 + S, the recognition sequence for N-linked glycosylation [Asn-X-Thr] within the preS2 + S ORF was mutated. The clone pUC13preS2S (Figure 1) served as the starting material for this construction.

To reconstruct the 5' portion of the preS2 + S ORF, a pair of oligonucleotides was synthesized to reconstitute the ORF from BamHI upstream to the ATG through a 10 bp NTL and a HindIII site to an EcoRI compatible terminus. The sequence of this oligonucleotide, which contains an A to C mutation (at base 31) and a T to A mutation (at base 33) and which would result in an aa change at position 4 of the S2 protein domain from Asn to Gln, is:

AAT TCA AGC TTA CAA AAC AAA ATG CAG TGG CAA TCC
    GT TCG AAT GTT TTG TTT TAC GTC ACC GTT AGG

ACT GCC TTC CAC CAA GCT CTG CAG
TGA CGG AAG GTG GTT CGA GAC GTC CTAG

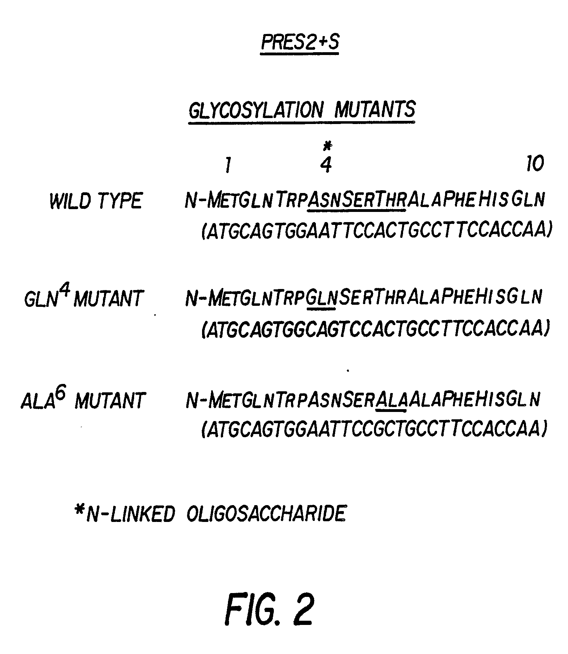

This synthetic oligonucleotide pair was ligated into pUC19 which had been digested previously with EcoRI and BamHI. The resultant plasmid was digested with BamHI and SalI and subsequently ligated with the 0.8 kbp BamHI to SalI fragment digested and purified from pUC13preS2S to create the plasmid pUC19 preS2SΔG-1 which, as a HindIII fragment, contains the preS2 + S ORF with Gln substituted for Asn at positon 4 (see figure 2). This ORF was used to create a yeast expression vector in an analogous fashion as described earlier.

In an analogous fashion the 0.8 kbp HindIII fragment was isolated from pUC13preS2S and ligated into a pUC19 vector in which the EcoRI and BamHI sites had been previously destroyed. The resultant vector was digested with EcoRI and BamHI and ligated with a pair of synthetic oligonucleotides which was designed to recreate the preS2 + S envelope ORF from EcoRI to BamHI with an A to G mutation (at base +7 of the oligonucleotide) which results in an amino acid interchange from Thr to Ala at amino acid +6 of the preS2 domain.

The sequence of this oligonucleotide is:

ATT TCC GCT GCC TTC CAC CAA GCT CTG CAA
    GG CGA CGG AAG GTG GTT CGA GAC GTT CTAG

This construction resulted in the creation of pUC19preS2SΔG-2 which contains the ORF as a HindIII fragment with Ala substituted for Thr at amino acid 6 of the preS2 domain (see figure 2). This ORF was used to create a yeast expression vector in an analogous fashion as described earlier.

PreS2 + S antigen expression was evaluated as described previously and was shown to be equivalent in productivity to that obtained with transformants described above. Clones of both mutants were preserved as frozen stocks for further evaluation. Immunoblot analysis developed with either anti-HBs (p24) sera or anti-preS2 sera detected a single major species with a molecular weight of ca. 30-kD which is consistent with that predicted for the non-glycosylated translation product of the preS2 + S ORF.

In order to further define and produce a specific glycosylation pattern of preS2 + S proteins by post-translational manipulations, the hyperglycosylated preS2 + S proteins prepared from yeast transformed with the plasmid pYGpreS2S-1 described earlier were deglycosylated enzymatically as described below:

The antigen preparation (purified by either immune affinity or hydrophobic interaction chromatography) was denatured by heating at 100°C for 5 minutes in 0.5% sodium dodecyl sulfate (SDS) and 0.2M β-mercaptoethanol, and then cooled in an ice bath. The enzymatic deglycosylation was initiated by addition of N-glycanase (peptide-N-glycosidase F, peptide-N⁴[N-acetyl-β-glucosaminyl]asparagine amidase), and the mixture was incubated at 37°C for 18 hrs. A control experiment was carried out under the same conditions, except that enzyme was not added. Release of oligosaccharide from protein was detected by a changed mobility of protein subunits on SDS-PAGE (silver stain analysis). Immunoblot analysis using two different antibodies against either the S antigen or against the preS2 domain also were used for detection of the deglycosylation reaction.

A critical feature of this invention is that the nonhyperglycosylated preS2 + S protein should contain and display important peptide domains and mimic conformational epitopes of infectious virions and of vaccine preparations with proven immunogenic efficacy (e.g., recombinant HBsAg). Therefore, we have studied the immunogenic potency of the nonhyperglycosylated preS2 + S protein in vivo as well as its antigenic profile in vitro. To this end, the preS2 + S ORF was expressed in yeast cells with the mnn9 phenotype, and the nonhyperglycosylated preS2 + S product was purified by either immune affinity or hydrophobic interaction chromatography.

For in vivo potency determinations, the nonhyperglycosylated preS2 + S preparation was adsorbed to alum, and groups of mice were injected with graded quantities of antigen. After six weeks, the mouse sera were assayed for anti-HBs antibody (AUSAB®) and anti-preS2 antibody [according to Neurath, J. Med. Virol., 17, 119-121, (1985)]. The results of such experiments indicated that the preS2 + S preparation was equally as effective as the HBsAg control preparation in inducing an anti-HBs antibody response (the effective immunizing dose was 0.34 μg for preS2 + S as compared to 0.25 μg for the HBsAg control). In addition, the preS2 + S preparation demonstrated at potent (effective immunizing dose of 0.14 μg) ability to induce a concommitant antibody response specific for the preS2 domain.

For determination of an in vitro antigenic profile, preparations of HBsAg, of hyperglycosylated preS2 + S and nonhyperglycosylated preS2 + S were analyzed using five different monoclonal antibodies (McAbs) to HBsAg. McAbs 1, 2, & 3 recognize three sites on the a conformational epitope on the surface of the 22 nm HBsAg particle or the infectious virion. McAbs 4 and 5 recognize the d epitope of HBsAg (McAb 4 recognizes a sequence of d and McAb 5 recognizes a conformation of d).

The results of this monoclonal analysis of preS2 + S antigens are significant on several levels. First, in general the nonhyperglycosylated antigen has a specific reactivity with the five McAbs several-fold higher than the hyperglycosylated antigen. This result argues that the hyperglycosylation is interfering with the immune binding of antibody. Second, McAb 1 has been shown to be a biologically important antibody in that it is protective in chimpanzees to live HBV challenge. This antibody binds to the core glycosylated preS2 + S six-fold better than to the hyperglycosylated preS2 + S antigen. McAb 1 reactivity to hepatitis B antigens has been strongly predictive and correlated to immune response in humans.

For recombinant proteins, the qualitative and quantitative glycosylation patterns are a function of and largely dependent upon the host cell species and within a species upon the cell line. It is thus obvious to those skilled in the art that the selection of a host strain extends to species and cell lines other than S. cerevisiae for which mutations in enzymes in the glycosylation pathway may be identified. It is also obvious to those skilled in the art that selection of host strains of S. cerevisiae extends to all strains in which mutations in enzymes of the glycosylation pathway may be identified. It is further obvious to those skilled in the art that, for limitation of glycosylation by post-translational modification, the selection of envelope protein extends to the protein products resulting from natural infection, from transgenic animals, and from recombinant hosts cells of species other than S. cerevisiae. It is further obvious that controlling limitation of glycosylation by altering, at the DNA level, the translated glycosylation signals extends to mutagenesis which results in deletion of all or part of the recognition sequence as well as to that which prevents glycosylation by amino acid substitution. It is also obvious to those skilled in the art that expression of such mutants extends, but is not limited, to species and cells derived from, but not limited to, mammals, insects and other fungi.

The genus Saccharomyces is composed of a variety of species. S. cerevisiae is most commonly used as a host for the recombinant DNA-mediated expression of a variety of foreign polypeptides. However, the distinctions among other species of the genus Saccharomyces are not always well-defined. Many of these species are capable of interbreeding with S. cerevisiae and are likely to possess promoters which are analogous or identical to promoters in S. cerevisiae. Therefore, it will be obvious to those skilled in the art that, for the expression of preS2 + S polypeptides, the selection of a host strain extends to other species of the genus Saccharomyces, including, but not limited, to carlsbergensis, diastaticus, elongisporus, kluyveri, montanus, norbensis, oviformis, rouxii, and uvarum.

Several yeast genera such as Candida, Hansenula, Pichia, and Torulopsis have been shown to contain similar metabolic pathways for the utilization of methanol as a sole carbon source for growth. The gene for alcohol oxidase, an enzyme which participates in this metabolic pathway, has been isolated from Pichia pastoris. The P. pastoris alcohol oxidase promoter has been isolated and shown to be susceptible to methanol induction of expression. Such an inducible promoter is useful for the expression of polypeptides in yeast. In particular, this promoter has been shown to be active on a plasmid for the inducible expression of the S domain in P. pastoris in particulate form. This observation highlights the ability of other yeast genera to function as hosts for the recombinant DNA-mediated expression of polypeptides in immunologically-active form. Therefore, it will be obvious to those skilled in the art that, for the expression of preS2 + S, the selection of a host strain extends to species from other genera of yeast from the Families Saccharomycetaceae and Cryptococcaceae, including, but not limited to Candida, Hansenula, Kluyveromyces, Pichia, Saccharomyceopsis, and Torulopsis.

The following examples illustrate the present invention without, however, limiting the same thereto. The disclosure of each reference mentioned in the following examples is hereby incorporated by reference.

## EXAMPLE I

### Cloning of HBV DNA in pBR322

HBV Dane particles (serotype adw) were isolated and purified from human plasma (carrier), and double-stranded DNA was synthesized by the endogenous polymerase in the Dane particles according to the methods of Landers et al., [J. Virology, 23, 368-376, (1977)] and Hruska et al., [J. Virology, 21, (1977)]. The DNA was isolated after digestion with Proteinase K in SDS followed by extraction with phenol/chloroform and ethanol precipitation. The HBV genomic DNA was digested with EcoRI, producing a single 3.2 kbp fragment, that was cloned into the EcoRI site of pBR322 to form pHBV/ADW-1 (Figure 1). The presence of the HBV DNA was confirmed by EcoRI digestion, Southern blot transfer to nitrocellulose, and hybridization with [32P]-labelled specific oligonucleotide probes.

## EXAMPLE II

### Cloning of the preS2 + S Gene into the pGAP-tADH-2 Expression Vector

As shown in Figure 1, plasmid pHBV/ADW-1 (described in Example I) was digested with EcoRI and AccI, and the 0.8 kbp fragment purified by preparative agarose gel electrophoresis.

To reconstruct the 5' portion of the preS2 + S ORF, a pair of oligonucleotides was synthesized which reconstitutes the ORF from the EcoRI site upstream to the ATG through a 10 bp NTL sequence to a HindIII terminus. The sequence of this oligonucleotide is:

AGCTTACAAAACAAAATGCAGTGG

ATGTTTTGTTTTACGTCACCTTAA

To reconstitute the 3' portion of the preS2 + S ORF, a second pair of oligonucleotides was synthesized which reconstitutes the ORF from the AccI site through the translational terminator to a HindIII terminus. The sequence of this oligonucleotide is:

ATACATTTAA

TGTAAATTTCGA

The plasmid pGAP-tADH-2 (see Fig. 1) containing the GAP491 promoter [Holland et al., J. Biol. Chem., 255:2596, (1980)] and the ADH1 transcriptional terminator in pBR322, has a unique HindIII cloning site into which the preS2 + S ORF described above was ligated, yielding pEGpreS2S-1 (Figure 1). The presence and orientation of HBV DNA was confirmed by restriction endonuclease analyses and Southern blot transfer. The expression cassette containing the preS2 + S ORF was removed from pEGpreS2S-1 by SphI digestion and isolated by preparative agarose gel electrophoresis. The cassette then was cloned into the shuttle vector pC1/1 (Beggs, supra; Rosenberg et al., supra) which had been digested previously with SphI to create a yeast expression vector (pYGpreS2S-1) which was then used to transform S. cerevisiae as described below.

## EXAMPLE III

### Transformation and Establishment of Seed Stocks for PreS2 + S Expression in Yeast "Wild Type" for Glycosylation

The resultant plasmid pYGpreS2S-1 (from Example II above) containing the expression cassette was used to transform S. cerevisiae strain CF42, (MATa/$\alpha$, adel⁻ leu2-04⁻, ura3⁻), which was created as follows:

A ura3 mutation in yeast strain 2150-2-3 (L. Hartwell, U. of Washington) was selected (Boeke et al., supra). The resulting strain (MATa, adel⁻, leu2-04⁻, ura3⁻, cir°) was diploidized by transforming with the plasmid YCp50-HO [Jensen et al., PNAS USA, 80: 3035-3039, (1983)]. A diploid strain was cured of the plasmid and designated CF42, (MATa/$\alpha$, adel⁻, leu2-04⁻, ura3⁻).

A transformed clone (pF403) was selected and established as a frozen stock (in 17% glycerol) for evaluation as described below.

## EXAMPLE IV

### Growth and Expression of the preS2-S Gene in Yeast "Wild-type" for Glycosylation

The clone pF403 of yeast containing the expression plasmid described in Example III as plated onto leu⁻ agar plates and incubated at 30°C for 2-3 days. These yeast were inoculated into 5-7 mL cultures of complex YEHD media, and the cultures were incubated at 30°C with aeration for 12-18 hrs. Flasks containing 50 mL complex YEHD media were inoculated from the above cultures to an A$^{600}$ of 0.1 and were incubated at 30°C with shaking (350 rpm) for 48-72 hrs to a final A$^{600}$ of 10-16. Triplicate samples of 10 A$^{600}$ units were aliquoted into tubes, and the yeast cells were pelleted at 2000xg for 10 min. The pellets either were assayed directly or stored at -70°C for future use as an internal reference standard for the evaluation of the controlled glycosylation clones described below in Example VII, VIII, IX and X (for these comparisons, values for clone pF403 were normalized to 1.0). At the time of assay, the pellets were resuspended in 0.4 mL of phosphate-buffered saline containing 2mM PMSF. Yeast cells were broken by: 1) the addition of 200-300 mg of washed glass beads (0.45 mm), 2) agitation on a vortex mixer for 15 min, 3) addition of TX-100 to 0.5% (v/v), 4) agitation on a vortex for 2 min, and 5) incubation at 4°C for 10 min. Cellular debris and glass beads were removed by centrifugation at 2000xg for 10 min. The clarified supernatant fluid was removed and assayed for protein [by the method of Lowry et al., J. Biol. Chem., 193, 265 (1951)] and by an RIA specific for preS2 + S [Hansson et al., supra. Machida et al., supra.].

## EXAMPLE V

### Yeast Transformation and Seed Establishment of PreS2 + S in a Circle ( + ) mnn9 Mutant Yeast

The resultant plasmid (pYGpreS2S-1) from Example II above containing the expression cassette was used to transform S. cerevisiae KHY-107 (cir⁺) which was constructed as follows:

The $\alpha$ mating type strain CZ5/LB347-1C (mnn 9⁻, SUCZ⁻) was mated with the a type strain 2150-2-3 (leu2⁻, adel⁻) by mixing the strains on a YEHD complete media plate. To select for diploids, the mated strains were replica plated onto leu⁻ minimal medium and containing 2% sucrose as the sole carbon source. After isolating single colonies, the diploids were sporulated, and asci were dissected by standard techniques. The KHY-107 strain was isolated as a single spore and characterized as cir⁺, adel⁺, leu2⁻, and mnn9⁻ (by Schiff stain technique).

Clones were selected on minimal medium (leu⁻ and containing 1M sorbitol), established as frozen stocks (in 17% glycerol) and evaluated as described below.

## EXAMPLE VI

### Yeast Transformation and Seed Establishment for PreS2-S in a Cir° mnn9 Mutant Yeast

The expression plasmid described in Example II above was used to transform S. cerevisiae strain KHY-107 (cir°) which was derived from strain KHY 107 (cir⁺) as described by Broach ["Methods in Enzymology", Vol 101, Part C, 307-325, (1983)]. Clones were selected, established as frozen stocks as described above in Example V, and evaluated for expression of preS2+S as described below in Example VIII.

## EXAMPLE VII

### Growth and Expression of the preS2+2 Gene in Circle + mnn9 Mutant Yeast

Clones of yeast containing the expression plasmid described in Example V were plated onto leu⁻ selective agar plates containing 1M sorbitol and incubated at 30°C for 2-3 days. These yeast were inoculated into 5-7 mL cultures of complex YEHDS and the cultures were incubated at 30°C with aeration for 12-18 hrs. Flasks containing 50 mL YEHDS media were inoculated from the above cultures (to an initial $A^{600}$ = 0.1) and were incubated at 30°C with shaking (350 rpm) for 48-72 hrs at a final $A^{600}$ of 10-16. Samples of 10 $A^{600}$ units were aliquoted into tubes, and the yeast cells were pelleted at 2000xg for 10 min. Samples either were assayed directly as described in Example IV or stored frozen at -70°C. Cellular debris and glass beads were removed by centrifugation at 2000xg for 10 min. The clarified supernatant fluid was removed and assayed for protein and by an RIA specific for preS2+S, as described previously.

Five clones were evaluated in parallel and compared to an equivalent cell pellet from clone pF403 (see Example IV above) which was normalized to a value of 1.0 for reference. Typical relative values of antigen productivity for the five clones were:

| Clone | Relative μg preS2+S/ml[1] | Relative units PreS2+S/units Protein[1] |
|---|---|---|
| a | 1.6 | 0.7 |
| b | 1.4 | 0.25 |
| c | 1.2 | 0.5 |
| d | 1.5 | 0.3 |
| e | 1.3 | 0.2 |
| pF403 | 1.0 | 1.0 |

(1) Ellis et al., (1987) In "Viral Hepatitis and Liver Disease" A. Zuckerman (ed), New York: Alan R. Liss Inc. p. 1079. and Kniskern, et al., (1988) Hepat logy, 8, 82-87.

Immunoblot analysis developed with rabbit anti-HBs (p24) or McAb to the preS2 domain detected a single major species with a molecular weight of ca. 34-kD. Clone "a" above, hereinafter referred to as Clone 14007-230-1A, was selected for further development.

EXAMPLE VIII

Growth and Expression of the preS2 + 2 Gene Cir° mnn9 Mutant Yeast

Clones of yeast containing the expression plasmid described in Example VI were plated onto leu⁻ selective agar plates containing 1M sorbitol and incubated at 30°C for 2-3 days. These yeast were inoculated into 5-7 mL of YEHDS medium and the cultures were incubated at 30°C with aeration for 12-18 hrs. Flasks containing 50 mL complex YEHDS medium were inoculated from the above cultures (to an initial $A^{600}$ = 0.1) and were incubated at 30°C with shaking (350 rpm) for 48-72 hrs to a final $A^{600}$ of 10-16. Triplicate samples of 10 $A^{600}$ units were aliquoted into tubes, and the yeast cells were pelleted at 2000xg for 10 min. Samples either were assayed directly as described previously or stored frozen at -70°C. Cellular debris and glass beads were removed by centrifugation at 2000xg for 10 min. The clarified supernatant fluid was removed and assayed for protein and preS2 + S antigen as described previously.

Five clones were evaluated in parallel and compared to clone pF403 (see Example IV above) which was normalized to a value of 1.0 for reference. Typical relative values of antigen productivity for the five clones were:

| Clone | Relative μg preS2 + S/ml* | Relative units PreS2 + S/units Protein* |
|---|---|---|
| a | 2.1 | 1.2 |
| b | 1.7 | 1.1 |
| c | 1.65 | 1.0 |
| d | 2.0 | 1.2 |
| e | 2.0 | 1.1 |
| pF403 | 1.0 | 1.0 |
| 14007-230-1A (from Example VII) | 1.8 | 1.0 |

* See Example VII

Immunoblot analysis (see Example VII) detected a single major band with a molecular weight of ca. 34-kD. Clone "a" above, hereinafter referred to as Clone 14007-284-1A, was selected for further development.

EXAMPLE IX

Additional Studies of Growth of the S. Cerevisiae (mnn9⁻) Producing preS2 + S in Shake Flasks

The frozen stock culture 14007-230-1A (Example VII above) was inoculated onto leu⁻ plates containing 1M sorbitol. The plates were incubated inverter at 28°C for 2-3 days. Seed cultures either were established as described in Example VII above or the growth on the plates was resuspended in YEHDS medium, and the resuspended growth was transferred into a .2-L Erlenmeyer flask containing 500 mL of YEHDS. The flask was incubated at 28°C and 350 rpm in a controlled environment shaker incubator, for 18-22 hrs.

An inoculum (5% (v/v)) from the seed flask was transferred into a 250-mL or 2-L flask containing 50-mL or 500-mL of YEHDS, respectively. The production flasks then were incubated as described above for 40-46 hrs. An optical density of 8.0 $A^{660}$ units typically was obtained. The cells from the flasks were harvested by centrifuging the contents of the flasks in 500-mL centrifuge bottles for 10 min at 1300xg. The supernatant was decanted and the cell pellet resuspended in 50-100 mL of a buffered salt solution.

Aliquots (0.6 mL) of 20% washed cell slurries were broken using glass beads (0.45-0.52 mm) in 1.5-mL Eppendorf tubes. PMSF (6.5 μl of 200 mM stock) was added as a protease inhibitor. Aliquots were removed from the tubes after breakage and frozen at -70°C for immunoblot analysis. Triton X-100 was added to the remaining sample in the tubes to a final concentration of 0.5%, and the samples were briefly mixed and incubated at 4°C for 20-40 min. The cell debris was removed by centrifugation and the clarified cell extract assayed for preS RIA and Lowry protein.

A typical value obtained was 15.65 μg preS2 + S/mL of fermentation broth and 0.04 mg of preS2 + S/mg of total protein.

## EXAMPLE X

## Large Scale Growth of S. Cerevisiae (mnn9⁻) Producing preS2 + S in Fermentors

The frozen stock culture 14007-230-1A was inoculated onto leu⁻ plates containing 1M sorbitol. The plates were incubated inverted at 28°C for 2-3 days. The growth on the plates was resuspended in YEHDS and the resuspended growth was transferred into 2-L Erlenmeyer flask containing 500 mL of YEHDS. The flask was incubated at 28°C and 350 rpm in a controlled environment shaker incubator for 18-22 hrs. These seed cultures then were used to inoculate the production stage vessels.

An inoculum (1-5% v/v) from one or more flasks was transferred into 16-L or 250-L fermentors containing 10-L or 200-L of YEHDS, respectively. The 16-L fermentors were operated at 500 rpm, 5 L/min air, and 28°C. The 250-L fermentors were operated at 160 RPM, 60 L/min air and 28°C. The fermentors were harvested 40-46 hrs after inoculation with the seed culture. Optical density values of 15.0 A⁶⁶⁰ units typically were obtained. Harvesting consisted of concentrating the cells using a hollow fiber filtering device followed by washing the cells in buffered salt solutions. Cell slurries were assayed as described below or stored frozen at -70°C for further processing and analysis.

Small samples (0.6 mL) of 20% washed cell slurries were broken using glass beads (0.45-0.52 mm) in 1.5-mL Eppendorf tubes. PMSF (6.5 μl of 200 mM stock) was added as a protease inhibitor. Aliquots were removed from the tubes after breakage and frozen at -70°C for immunoblot analysis. Triton X-100 was added to the remaining sample in the tubes to a final concentration of 0.5%, and the samples were mixed briefly and incubated at 4°C for 20-40 min. The cell debris was removed by centrifugation and the clarified cell extract assayed for preS RIA and Lowry protein. An average value for antigen productivity was 8.4 μg of preS2 + S/mL of fermentation broth and 0.025 mg preS2 + S protein/mg of total protein.

## EXAMPLE XI

## Cloning of the preS2 + S Gene in which the Recognition Sequence for N-linked Glycosylation (Asn-X-Thr) is Changed to Gln-X-Thr Thereby Preventing Glycosylation

The PreS2 + S ORF which served as the starting material for this construction is contained in the subclone (pUC13preS2S) described in Example II above and identified in Figure 1. To reconstruct the 5′ portion of the preS2 + S ORF, a pair of oligonucleotides was synthesized which reconstitutes the ORF from BamHI upstream to the ATG through a 10 bp NTL, HindIII site to an EcoRI compatible terminus. The sequence of this oligonucleotide, which contains an A to C interchange (at base 31) and a T to A interchange (at base 33) and which results in an inputed amino acid change at position 4 of the preS2 domain from Asn to Gln, is:

AAT TCA AGC TTA CAA AAC AAA ATG CAG TGG CAA TCC
    GT TCG AAT GTT TTG TTT TAC GTC ACC GTT AGG

ACT GCC TTC CAC CAA GCT CTG CAG
TGA CGG AAG GTG GTT CGA GAC GTC CTAG

This synthetic oligonucleotide was ligated into pUC19 which had been digested previously with EcoRI and BamHI. The resultant plasmid was digested with BamHI and SalI and subsequently ligated to the 0.8 kbp BamHI to SalI fragment previously purified from pUC13preS2S to create the plasmid pUC19preS2SΔG1 which, as a HindIII fragment, contains the preS2+S ORF with Gln substituted for Asn at aa position 4 (see figure 2). This ORF [hereafter called the preS2S (Gln⁴) mutant] was used to create a yeast expression vector in a fashion analogous to that described in Example II above. This plasmid was used to transform yeast strain CF42 as described above in Example III. Clones were selected, established as frozen stocks as described in Example V and evaluated for expression of preS2+S as described below in Example XII.

## EXAMPLE XII

Growth and Expression of the preS2S(Gln⁴) Mutant ORF in S. cerevisiae

Yeast clones containing the recombinant plasmid established in Example XI above were evaluated for antigen expression as described in Example IV. Five clones were evaluated in parallel and compared to cell pellets from clone pF403 (see Example IV) which was normalized to a value of 1.0 for reference. Typical values of antigen productivity for the six clones were:

| Clone | Relative μg preS2+S/ml[1] | Relative units PreS2+S/units Protein[1] |
|---|---|---|
| a | 1.0 | 0.8 |
| b | 0.75 | 0.8 |
| c | 1.1 | 1.4 |
| d | 1.1 | 1.6 |
| e | 0.94 | 0.8 |
| pF403 | 1.0 | 1.0 |

(1) See Example VII

Immunoblot analysis detected a single major species with a molecular weight of ca. 31-kD which is consistent with that predicted for the nonglycosylated translation product of the preS2+S ORF. Clone d, hereafter referred to as clone 14501-35-Cl, was selected for further development.

## EXAMPLE XIII

Cloning of the preS2+S Gene in which the Recognition Sequence for N-linked Glycosylation (Asn-X-Thr) is Changed to Asn-X-Ala, Thereby Preventing Glycosylation

The preS2+S ORF, which served as the starting material for this construction is contained in the subclone (pUC13preS2S) described in Example II above and identified in Figure 1. The 0.8 kbp HindIII fragment was isolated from pUC13preS2S and ligated into a pUC19 vector in which the EcoRI and BamHI sites had been destroyed previously. The resultant vector was digested with EcoRI and BamHI and ligated with a synthetic oligonucleotide which was designed to recreate the preS2 + S envelope ORF from EcoRI to BamHI with an A to G interchange (at base +7 of the oligonucleotide) which results in an amino acid interchange from Thr to Ala at amino acid +6 of the preS2 domain.

The sequence of this oligonucleotide is:

ATT TCC GCT GCC TTC CAC CAA GCT CTG CAA

GG CGA CGG AAG GTG GTT CGA GAC GTT CTAG

This construction resulted in creation of plasmid pUC19preS2 + SΔG-2 which contains the preS2S ORF as a HindIII fragment with Ala substituted for Thr at amino acid 6 of the preS2 domain (see figure 2). The ORF [hereafter called the preS2S (Ala$^6$) mutant] was used to create a yeast expression vector in a fashion analogous to that described in Example II above. This plasmid was used to transform yeast strain CF42 as described above in Example III. Clones were selected, established as frozen stocks as described in Example V and evaluated for expression of preS2 + S as described below in Example XIV.

EXAMPLE XIV

Growth and Expression of the preS2 + S (Ala$^6$) Mutant ORF in S. cerevisiae

Yeast clones containing the recombinant plasmid established in Example XIII above were evaluated for antigen expression as described in Example IV. One of three clones evaluated gave high-level expression of the preS2 + S polypeptide. Typical values for antigen expression for this clone relative to pF403 (normalized to 1.0) were: 1.3 μg preS2 + S/mL and 0.6 units preS2 + S protein/unit total yeast protein.

Immunoblot analysis detected a single species with a molecular weight of ca. 31-kD, which is consistent with that predicted for the nonglycosylated translation product of the preS2 + S ORF.

This clone, hereafter referred to as 14501-34-A1, was selected for further development.

EXAMPLE XV

Removal of N-linked Glycosylation by Enzymatic Digestion with N-glycanase

Hepatitis B preS2 + S proteins prepared from yeast transformed with the plasmids described above in Examples IV, VII and VIII, where enzymatically deglycosylated as follows:

The antigen preparation (purified by either immunoaffinity or hydrophobic interaction chromatography) was denatured by heating at 100°C for 5 min in 0.5% SDS and 0.2M β-mercaptoethanol then cooled in an ice bath. To the denatured protein (300 μg in 0.5 mL) the following were added: a) 0.25 mL of 1M sodium phosphate, pH 8.6, b) 0.15 mL of 0.1M 1,10 phenanthroline hydrate in methanol and c) 0.25 mL of 7.5% NP-40® [polyoxyethylene (9)octaphenol]. The enzymatic deglycosylation was initiated by addition of 10 units of N-glycanase (peptide-N-glycosidase F, peptide-N$^4$ [N-acetyl-β-glucosaminyl]asparagine amidase) to the above mixture, and the mixture was incubated at 37°C for 12-24 hrs. A control experiment was carried out under the same conditions except that no enzyme was added. Release of oligosaccharide from protein was detected by the differential mobility or protein subunits on SDS-PAGE with silver stain detection. Immunoblot analysis also was performed as described in Example VII above. Such SDS-PAGE analysis revealed a single major species with a molecular weight of ca. 31-kD which migrated at the same position on the gel as the nonglycosylated species described in Examples XII & XIV above and which is consistent with a molecular weight predicted for the polypeptide translation product of the preS2 + S ORF.

EXAMPLE XVI

15

Removal of N-linked Glycosylation by Enzymatic Digestion with Endoglycosidase H (Endo H)

Hepatitis B preS2 + S proteins prepared from yeast transformed with the plasmids described above in Examples IV, VII and VIII were enzymatically treated as follows:

The antigen preparation first was denatured by heating 100 $\mu$l of protein containing 40 $\mu$g Lowry protein/mL in a boiling water bath with 0.48 $\mu$l of 1% SDS, for 10 min. After heating, the material was cooled on ice, and 20 $\mu$l was placed in a reaction vial to which the following additions were made: a) 15 $\mu$l of 50 mM sodium phosphate buffer pH 6.0, and b) 25 $\mu$l of Endo H (2000 mU/ml). The mixture then was incubated for 16 hrs at 37° C. A control was run under the same conditions without Endo H. Release of oligosaccharide from protein was detected by the differential mobility of polypeptide subunits on SDS-PAGE with silver stain detection. Immunoblot analysis also was performed as described in Example VII above. Such SDS + PAGE analysis revealed a single major species with a molecular weight of ca. 31-kD which migrated at the same position on the gel as the nonglycosylated species described in Examples XII & XIV above and which is consistent with a molecular weight predicted for the polypeptide translation product of the preS2 + S ORF.

## EXAMPLE XVII

Digestion of PreS2 + S Polypeptides with Endo F

Hepatitis B preS2 + S prepared from yeast transformed with the plasmids described above in Example IV, VII and VIII was enzymatically digested as follows:

The antigen preparation first was denatured by heating 100 $\mu$l of protein containing 40 $\mu$g/mL by Lowry Protein with 2.5 $\mu$l of 10% SDS and 0.725 $\mu$l of $\beta$ mercaptoethanol (1.43 M) for 3 min in a boiling water bath. The antigen preparation was cooled, 10 $\mu$l was placed in a reaction vial and the following additions were made: a) 7 $\mu$l of 0.44 M sodium acetate, pH 6.0, b) 3 $\mu$l of 100 mM 1,10 phenanthroline hydrate (in methanol), c) 5 $\mu$l of 7.5% NP-40, and d) 5 $\mu$l of Endo F (60 mU/ml). This mixture then was incubated for 16 hrs at 37° C. A control was run under the same conditions without Endo F. Release of oligosaccharide was detected as described in Examples XV and XVI. However, digestion with Endo F did not release appreciable oligosaccharide as indicated by the migration of the major species at ca. 34-kD both after and before enzyme treatment.

## EXAMPLE XVIII

## McAb Analysis of HB Antigens

The three HBsAg samples listed below were analyzed using five different McAb to HBsAg. 1, 2 and 3 recognize three sites on the a conformational epitope on the surface of the 22 nm HBsAg particle. McAb 4 and 5 recognize the d epitope of HBsAg, (McAb 4 recognizes a sequence on d and McAb 5 recognizes a conformation of d). The assay is performed by incubating 1 ng of antibody with graded dilutions of 0.1-1.0 ng antigen. Following incubation for two hours at room temperature, unbound anti-HBs McAb is detected in an AUSAB® assay. The counts per minute are plotted as HBsAg concentration, and a potency is derived from the slope; when compared to a standard HBsAg preparation, a relative potency is calculated. The results of these analyses are shown in the table below:

| Antigen | Relative Potency | | | | |
|---|---|---|---|---|---|
| | McAb-1 | McAb-2 | McAb-3 | McAb-4 | McAb-5 |
| HBsAg (S) | 1.0 | 1.0 | 1.0 | 1.0 | 1.0 |
| preS2 + S (hyperglycosylated) | 0.1 | 0.4 | 0.4 | 0.6 | 0.3 |
| preS2 + S (core glycosylated) | 0.6 | 0.7 | 0.9 | 0.6 | 0.8 |

EXAMPLE XIX

Mouse Potency Assay of Nonhyperglycosylated PreS2 + S Hepatitis B Proteins

Groups of 10 BALB/c mice were injected intraperitoneally with graded dilutions of alum-adsorbed HBsAg or preS2 + S. After six weeks, the sera were assayed for the presence of anti-HBs antibody (AUSAB®) and anti-preS2 antibody [Neurath, J. Medical Virology, 17:119-125, (1985)] The results of a typical experiment, which verifies the immunogenicity of preS2 + S preparations, are summarized below:

| Vaccine | Dosage (mcg) | Anti-HBs Response | | | Anti-preS2 Response | |
|---|---|---|---|---|---|---|
| | | Positive mice Total mice | Geometric mean Titer (Ausab titer) | $ED_{50}$ | Positive mice Total mice | $ED_{50}$ |
| HBsAg (S) | 2.0 | 10/10 | 1097 | | 0/10 | |
| | 0.67 | 9/10 | 854 | 0.25 mcg | 0/10 | |
| | 0.22 | 5/10 | 165 | | 0/10 | >2.0mcg |
| | 0.07 | 0/10 | 27 | | 0/10 | |
| preS2 + S (core glycosylated) | 2.0 | 10/10 | 1260 | | 10/10 | |
| | 0.67 | 8/10 | 386 | | 10/10 | |
| | 0.22 | 3/10 | 64 | 0.34 mcg | 8/10 | 0.14mcg |
| preS2 + S | 0.07 | 0/10 | 20 | | 1/10 | |

## Claims

1. A hepatitis B virus (HBV) protein comprising at least part of the preS domain contiguously linked to at least part of the S protein domain, in which glycosylation is minimized or eliminated and which displays major antigenic sites of the preS and S envelope proteins.

2. A protein according to Claim 1 wherein the preS domain comprises at least part of the preS1 region.

3. A protein according to Claim 1 wherein the preS domain comprises at least part of the preS2 region.

4. A protein according to Claim 3 additionally including at least part of the preS1 domain.

5. A protein according to Claim 1 wherein the glycosylation of the HBV polypeptide is limited by expression of the polypeptide in a yeast host cell that is defective for a gene in the glycosylation pathway.

6. A protein according to Claim 5 wherein the glycosylation of the HBV polypeptide is limited to not more than 15 sugar residues.

7. A protein according to Claim 5 wherein the host cell glycosylating defect is in the mnn9 gene.

8. A protein according to Claim 1 wherein glycosylation of the polypeptides is prevented in vivo by growth of the recombinant cells in the presence of an exogenous agent that inhibits normal glycosylation.

9. A protein according to Claim 8 wherein the agent is tunicamycin.

10. A protein according to Claim 1 wherein the glycosylation of the polypeptides is eliminated by altering DNA codons that code for at least one amino acid in the amino acid recognition sequences for N-linked glycosylation to an amino acid that is not capable of being glycosylated or that is not part of the amino acid recognition sequences for N-linked glycosylation.

11. A protein according to the Claim 10 wherein the N-linked glycosylation of the preS2 domain is eliminated by altering at least one of the amino acids of the recognition sequence at residues 4-6 in the preS2 domain to one not capable of being glycosylated or one that is not part of amino acid recognition sequences for N-linked glycosylation.

12. A protein according to Claim 11 wherein the amino acid at position 4 of the preS2 domain is any amino acid except Asn.

13. A protein according to Claim 11 wherein the amino acid at position 4 is Gln.

14. A protein according to Claim 11 wherein the amino acid at position 6 is any amino acid except Thr or Ser.

15. A protein according to Claim 11 wherein the amino acid at position 6 is Ala.

16. A protein according to Claim 1 wherein the glycosylation is minimized or eliminated post-translationally by chemical or enzymatic removal of all or part of the N-linked glycan.

17. A protein according to Claim 16 wherein the enzyme is N-glycanase. Endo D, Endo F, Endo H or Glycopeptidase A.

18. A protein according to Claim 16 wherein the chemical agent is anhydrous trifluoromethane sulfonic acid or anhydrous hydrogen fluoride.

19. An expression cassette comprising at least a promoter sequence that is active in yeast, an ORF encoding at least part of the HBV preS domain linked to all or part of the S domain, and a suitable transcriptional termination sequence that is active in yeast, wherein nucleotides in the ORF have been altered in order to eliminate N-linked glycosylation in the HBV protein product.

20. An expression cassette according to Claim 19 wherein the DNA encodes the HBV preS1 + preS2 + S polypeptide.

21. An expression cassette according to Claim 19 wherein the DNA encodes the HBV preS2 + S polypeptide.

22. An expression cassette according to Claim 19 wherein the DNA encodes polypeptides displaying HBV antigenicity.

23. An expression cassette according to Claim 19 wherein the DNA sequence encoding the amino acid at position 4 of the preS2 domain is not AAT or AAC.

24. An expression cassette according to Claim 19 wherein the DNA sequence encoding the amino acid at position 4 of the preS2 domain is CAA or CAG.

25. An expression cassette according to Claim 19 wherein the DNA sequence encoding the amino acid at position 6 of the preS2 domain is not AGT, AGC, ACT, ACC, ACA or ACG.

26. An expression cassette according to Claim 19 wherein the DNA sequence encoding the amino acid at position 6 of the preS2 domain is GCT or GCC or GCA or GCG.

27. A host cell of a species of yeast containing an expression cassette according to Claim 19.

28. A host cell of a species of yeast according to Claim 27 wherein the host cell species contains in biochemically active form all enzymes required for glycosylation and mannoprotein biosynthesis.

29. A host cell of a species of yeast according to Claim 27 wherein the strain is CF42.

30. A host cell of a species of yeast wherein the host cell contains a defective gene or genes encoding enzymes in the glycosylation pathway and containing an expression cassette comprising at least a promoter sequence, an ORF encoding all or part of the HBV preS domain linked to all or part of the S domain, and a suitable transcription termination sequence.

31. A host cell of a species of yeast according to Claim 30 wherein the defect is in the mnn9 gene.

32. A species of yeast according to Claim 31 wherein the strain is KHY-107.

33. A process for obtaining the preS2 + S polypeptide comprising:

a. transforming host cells from a species of yeast with DNA containing an expression cassette of Claim 25,

b. culturing the transformed cells, and

c. recovering the polypeptide from the resulting cultured cells or growth medium.

34. A process according to Claim 33 wherein the host cell is S. cerevisiae strain CF42.

35. A process for obtaining the preS2 + S polypeptide comprising:

a. transforming host cells from a species of yeast wherein the host cell contains a defective gene or genes encoding enzymes in the glycosylation pathway with DNA containing an expression cassette comprising at least a promoter sequence, an ORF encoding all or part of the preS domain linked to all or part of the S domain, and a suitable transcription termination sequence,

b. culturing the transformed cells, and

c. recovering the polypeptide from the resulting cultured cells or growth medium.

36. A process according to Claim 35, wherein the host cell is defective in the mnn9 gene.
37. A process according to Claim 36 wherein the host cell is S. cerevisiae strain KHY-107.
38. A diagnostic reagent comprising the polypeptide of Claim 1.

FIG. 1

PRES2+S

GLYCOSYLATION MUTANTS

                            *

              1             4                      10

WILD TYPE     N-METGLNTRP<u>ASNSERTHR</u>ALAPHEHISGLN

                   (ATGCAGTGGAATTCCACTGCCTTCCACCAA)

GLN$^4$ MUTANT    N-METGLNTRP<u>GLN</u>SERTHRALAPHEHISGLN

                   (ATGCAGTGGCAGTCCACTGCCTTCCACCAA)

ALA$^6$ MUTANT    N-METGLNTRPASNSER<u>ALA</u>ALAPHEHISGLN

                   (ATGCAGTGGAATTCCGCTGCCTTCCACCAA)

*N-LINKED OLIGOSACCHARIDE

# FIG. 2